(19) Europäisches Patentamt
European Patent Office
Office européen des brevets

(11) **EP 3 318 995 A1**

(12) **EUROPEAN PATENT APPLICATION**

(43) Date of publication:
**09.05.2018 Bulletin 2018/19**

(51) Int Cl.:
**G06F 19/24** *(2011.01)* **G01N 33/48** *(2006.01)*
**C12Q 1/00** *(2006.01)*

(21) Application number: **17192958.1**

(22) Date of filing: **25.04.2007**

(84) Designated Contracting States:
**AT BE BG CH CY CZ DE DK EE ES FI FR GB GR HU IE IS IT LI LT LU LV MC MT NL PL PT RO SE SI SK TR**

(30) Priority: **01.05.2006 US 381104**

(62) Document number(s) of the earlier application(s) in accordance with Art. 76 EPC:
**07761281.0 / 2 016 405**

(71) Applicant: **Provista Diagnostics, LLC Phoenix AZ 85016 (US)**

(72) Inventor: **RANDALL, Grimes, F. Scottsdale, AZ Arizona 85254 (US)**

(74) Representative: **Mummery, Thomas Zack Reddie & Grose LLP The White Chapel Building 10 Whitechapel High Street London E1 8QS (GB)**

Remarks:
•This application was filed on 25-09-2017 as a divisional application to the application mentioned under INID code 62.
•Claims filed after the date of filing of the application (Rule 68(4) EPC)/ after date of receipt of divisional application

(54) **METHODS AND APPARATUS FOR IDENTIFYING DISEASE STATUS USING BIOMARKERS**

(57) Methods and apparatus for identifying disease status according to various aspects of the present invention include analysing the levels of one or more biomarkers. The methods and apparatus may use biomarker data for a condition-positive cohort and a condition-negative cohort and select multiple relevant biomarkers from the plurality of biomarkers. The system may generate a statistical model for determining the disease status according to differences between the biomarker data for the relevant biomarkers of the respective cohorts. The methods and apparatus may also facilitate ascertaining the disease status of an individual by producing a composite score for an individual patient and comparing the patient's composite score to one or more thresholds for identifying potential disease status.

FIG.6

EP 3 318 995 A1

**Description**

FIELD OF THE INVENTION

The invention relates generally to methods and apparatus for identifying disease status in a patient, and more particularly to identifying disease status in a patient according to levels of one or more biomarkers.

BACKGROUND OF THE INVENTION

Biomarkers are used in medicine to help diagnose or determine the presence, absence, status and/or stage of particular diseases, Diagnostically useful biomarkers have been identified using measured levels of a single biomarker obtained from a statistically significant number of disease-negative and disease-positive subjects in a population and establishing a mean and a standard deviation tor the disease negative and positive stales. If the measured biomarker concentrations for the disease-positive and -negative states were found to have widely separated Gaussian or nearly Gaussian distributions, the biomarker was considered useful for predicting instances of the disease. Subsequent patients could be considered disease-positive if the patient's biomarker concentration was above (or, in some cases, below) a cut point generally defined as a biomarker concentration that is between the disease-positive and disease-negative means and two to three standard deviations away from the disease slate negative mean.

While conventional methods have produced clinically useful biomarkers, their application to determining a variety of disease statuses in subjects is limited for at least five reasons. First, these methods presume a normal, Gaussian data distribution in the population, where all measured biomarker concentrations are roughly distributed symmetrically above and below a mean and take the shape of a bell curve. In such cases, approximately 68% of the data is within one standard deviation of the mean, 95% of the data is within two standard deviations of the mean, and 99.7% of the data is within three standard deviations of the mean in either the disease-positive or -negative cohort. This assumption, however, only holds true for a fraction of all potential biomarkers. Human biochemistry is a complex system in which many components serve multiple functions and are themselves regulated by a variety of other components. As such, it is common to find biomarkers that display non-Gaussian distributions, which include values that lie substantially apart (at the fur high end and/or far low end of the distribution) from the bulk of the values, and may span several orders of magnitude.

Second, traditional methods rely on the analysis of a single biomarker to indicate a disease state. Given the complex interaction of human biochemistry, however, the interaction of multiple markers often have a bearing on the presence or absence of disease. Instead of integrating multiple statistically significant markers, single marker models rely on the ideal (or nearly ideal) performance of a single marker, which may result in a less accurate diagnosis of a disease state than integrating multiple biomarkers.

Third, conventional methods rely exclusively on large differences between disease-negative and disease-positive populations, and disregard all information when the distributions of the disease-negative and disease-positive populations overlap to any significant degree. In traditional single marker models, differences between the means of the negative disease stale and the positive disease state that are less than one and one-half to two standard deviations ore considered to have little or no value, even when these differences are found to be persistent and reproducible.

Fourth, the traditional single marker methods are often confounded by biodiversity and the presence of sub-groups in the disease-negative or disease-positive populations. Given the complexity of human biochemistry, many factors can affect the measured concentration of a given biomarker, such as a patient's demographic characteristics, family history and medical history. All of these factors may increase the potential marker's observed variability and standard deviation, masking or obscuring the relationship to the disease state.

Finally, despite increasing understanding of biomarkers and availability of convenient biomarker assays (e.g., immunohistochemistry assays) to detect and quantify expression of specific biomarkers associated with a disease, traditional analyses often fail to sufficiently differentiate the disease-negative and disease-positive statuses to permit reliable diagnosis of diseases.

SUMMARY OF THE INVENTION

Methods and apparatus for identifying disease status according to various aspects of the present invention include analyzing the levels of one or more biomarkers. The methods and apparatus may use biomarker data for a condition-positive cohort and a condition-negative cohort and automatically select multiple relevant biomarkers from the plurality of biomarkers. The system may automatically generate a statistical model for determining the disease status according to differences between the biomarker data for the relevant biomarkers of the respective cohorts. The methods and apparatus may also facilitate ascertaining the disease status of an individual by producing a composite score for un individual patient and comparing the patient's composite score to one or more thresholds for identifying potential disease status.

BRIEF DESCRIPTION OF THE DRAWING FIGURES

A more complete understanding of the present invention may be derived by referring to the detailed description when considered in connection with the following illustrative figures. In the following figures, like reference numbers refer to similar elements and steps.

Figure 1 is a block diagram of a computer system.
Figure 2 is a flow chart of a process for identifying disease status.
Figure 3 is a flow chart of a process for controlling a range of values.
Figure 4 is a flow chart of a process for normalizing data.
Figure 5 is a flow chart of a process for classifying data according to cut points.
Figure 6 is a plot of cumulative frequencies of disease-positive and disease-negative biomarker concentrations.
Figure 7 is a flow chart of a process for establishing a disease status model.
Figure 8 is a flow chart of a process for identifying disease status in an individual.
Figure 9 is a plot of cumulative frequencies of breast cancer positive and breast cancer negative concentrations versus PSA concentration.
Figure 10 illustrates data scoring model for selecting one or more cut points.

Elements and steps in the figures are illustrated for simplicity and clarity and have not necessarily been rendered according to any particular sequence. For example, steps that may be performed concurrently or in different order are illustrated in the figures to help to improve understanding of embodiments of the present invention.

DETAILED DESCRIPTION OF THE INVENTION

The present invention is described partly in terms of functional components and various processing steps. Such functional components and processing steps may be realized by any number of components, operations and techniques configured to perform the specified functions and achieve the various results. For example, the present invention may employ various biological samples, biomarkers, elements, materials, computers, data sources, storage systems and media, information gathering techniques and processes, data processing criteria, statistical analyses, regression analyses and the like, which may carry out a variety of functions. In addition, although the invention is described in the medical diagnosis context, the present invention may be practiced in conjunction with any number of applications, environments and data analyses; the systems described are merely exemplary applications for the invention.

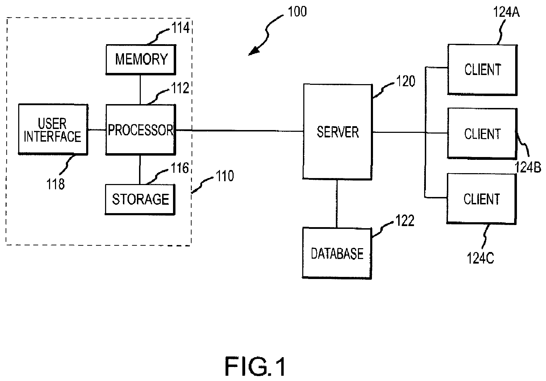

Methods and apparatus fur analyzing biomarker information according to various aspects of the present invention may be implemented in any suitable manner, for example using a computer program operating on the computer system. Referring to Figure 1, an exemplary biomarker analysis system 100 according to various aspects of the present invention may be implemented in conjunction with a computer system 110, for example a conventional computer system comprising a. processor 112 and a random access memory 114, such as a remotely-accessible application server, network server, personal computer or workstation. The computer system 110 also suitably includes additional memory devices or information storage systems, such as a mass storage system 116 and a user interface 118, for example a conventional monitor, keyboard and tracking device. The computer system 110 may, however, comprise any suitable computer system and associated equipment and may be configured in any suitable manner. In one embodiment, the computer system 110 comprises a stand-alone system. In another embodiment, the computer system 110 is part of a network of computers including a server 120 and a database 122. The database stores information that may be made accessible to multiple users 124A-C, such as different users connected to the server 120. In the present embodiment, the server 120 comprises a remotely-accessible server, such as an application server that may be accessed via a network, such as a local area network or the Internet.

The software required for receiving, processing, and analyzing biomarker information may be implemented in a single device or implemented in a plurality of devices. The software may be accessible via a network such that storage and processing of information takes place remotely with respect to users 124A-C. The biomarker analysis system 100 according to various aspects of the present invention and its various elements provide functions and operations to facilitate biomarker analysis, such as data gathering, processing, analysis, reporting and/or diagnosis. The present biomarker analysis system 100 maintains information relating to biomarkers and facilitates the analysis and/or diagnosis. For example, in the present embodiment, the computer system 110 executes the computer program, which may receive, store, search, analyze, and report information relating to biomarkers. The computer program may comprise multiple modules performing various functions or operations, such as a processing module for processing raw data and generating supplemental data and an analysis module for analyzing raw data and supplemental data to generate a disease status model and/or diagnosis information.

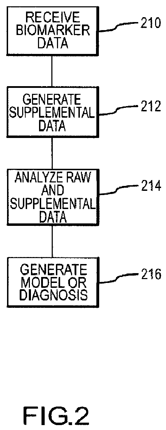

The procedures performed by the biomarker analysis system 100 may comprise any suitable processes to facilitate biomarker analysis and/or diagnosis. In one embodiment, the biomarker analysis system 100 is configured to establish a disease status model and/or determine disease status in a patient. Determining or identifying disease status may comprise generating any useful information regarding the condition of the patient relative to the disease, such as performing a diagnosis, providing information helpful to a diagnosis, assessing the stage or progress of a disease, identifying a condition that may indicate a susceptibility to the disease, identity whether further tests may be recommended, or otherwise assess the disease status, likelihood of disease, or other health aspect of the patient. Referring to Figure 2, in the present embodiment, the biomarker analysis system 100 receives raw biomarker data and subject data (210) relating to one or more individuals providing the biological samples from which the biomarker data is drawn. The biomarker analysis system 100 processes the raw data and subject data to generate supplemental data (212), and analyzes the raw data, subject data, and/or supplemental data (214) to establish a disease state model and/or a patient diagnosis (216).

The biomarker analysis system 100 may also provide various additional modules and/or individual functions. For example, the biomarker analysis system 100 may also include a reporting function, for example to provide information relating to the processing and analysis functions. The biomarker analysis system 100 may also provide various administrative and management functions, such as controlling access and performing other administrative functions.

The biomarker analysis system 100 suitably generates a disease status model and/or provides a diagnosis for a patient based on raw biomarker data and/or additional subject data relating to the subjects in the cohorts. The biomarker data may be acquired from any suitable biological samples containing measurable amounts of the biomarkers.

In accordance with various aspects of the invention, biomarker data are obtained and processed to establish a disease status model that incorporates data from a plurality of biomarkers, such as data from members of disease-negative and disease-positive cohorts or other condition-positive and/or -negative groups. The biological samples are suitably obtained from a statistically significant number of disease-positive and -negative subjects. Disease-positive and -negative cohorts may contain a sufficient number of subjects to ensure that the data obtained are substantially characteristic of the disease-negative and disease-positive states, such as statistically representative groups. For example, each cohort may have at least 30 subjects in each cohort. Each cohort may be characterized by several sub-cohorts, reflecting, for example, that the disease can exist in disease-positive individuals at various stages, or other demographic, behavioral, or other factors that may affect the biomarker levels in either disease-positive or -negative individuals.

The biomarker analysis system 100 may utilize any single or combination of biological materials from which the levels of potential biomarkers may be reproducibly determined. In the present embodiment, levels of all measured biomarkers are obtained from as few sample sources as possible, such as from a single, readily obtained sample. For example, sample sources may include, but are not limited to, whole blood, serum, plasma, urine, saliva, mucous, aspirates (including nipple aspirates) or tissues (including breast tissue or other tissue sample). Biomarker levels may vary from source-to-source and disease-indicating levels may be found only in a particular sample source. Consequently, the same sample sources are suitably used both for creating disease status models and evaluating patients. If a disease status model is constructed from biomarker levels measured in whole blood, then the test sample from a patient may also be whole blood. Where samples are processed before testing, all samples may be treated in a like manner and randomly collected and processed.

The biomarker analysis system 100 may analyze any appropriate quantity or characteristic. In the present case, a biomarker may comprise any disease-mediated physical trait that can be quantified, and in one embodiment, may comprise a distinctive biochemical indicator of a biological process or event. Many biomarkers are available for use, and the biomarker analysis system 100 provides an analytical framework for modeling and evaluating biomarker level data.

Raw biomarker levels in the samples may be measured using any of a variety of methods, and a plurality of measuring tools may be used to acquire biomarker level data. For example, suitable measuring tools may include, but are not limited to, any suitable format of enzyme-linked immunosorbent assay (ELTSA), radioimmunoassay (RIA), flow cytometry, mass spectrometry or the like. As biomarker levels may vary from method to method and from procedure to procedure, the biomarker analysis system 100 of the present embodiment uses consistent methods and procedures for creating disease status models as well as for evaluating patients. For example, if a disease status model is constructed from biomarker levels measured using a specified ELISA protocol, then the test sample from a patient should be measured using the same ELISA protocol.

The biomarker data, such as the raw biomarker levels and any other relevant data, are provided to the biomarker analysis system 100 for processing. One or more markers may be analyzed by the biomarker analysis system 100. The biomarker analysis system 100 may process the biomarker data to incorporate multiple markers, minimize potential impact of non-Gaussian distributions, and account for biodiversity. In the present embodiment, the biomarker analysis system 100 analyzes multiple biomarkers, assigns boundary values for the biomarker levels, generates normalized data bused on the raw data and potentially relevant biomarker-affecting factors, compares biomarkers to cut points, and/or reduces the range of raw and/or adjusted data values. The biomarker analysis system 100 may also adjust the data for disease-specific risk factors and analyze the data to generate the disease status model.

In one embodiment, the biomarker analysis system 100 may analyze multiple biomarkers to establish a disease status model and generate a diagnosis. Given the complex interaction of human biochemistry, multiple markers may have a relationship with the presence or absence of the disease state. Further, a single biomarker may not be associated exclusively with only one disease. While a single biomarker may provide useful information, diagnostic reliability may be improved by including a plurality of biomarkers, for example the most informative biomarkers. The biomarker analysis system 100 suitably integrates these multiple, less than ideal, but still statistically significant and informative biomarkers.

The biomarker analysis system 100 may assess whether a given biomarker is informative, such as according to a classification of not informative, informative, or highly informative, and whether it is productive to include the marker in the disease status model. For example, various biomarkers are associated with breast cancer and, when modeling characteristic biomarker levels and evaluating breast cancer in subjects, such markers may be highly relevant. In one particular example, up-regulated (elevated) and/or down-regulated (suppressed) levels in serum of prostate-specific antigen (PSA), tumor necrosis factor alpha (TNF-$\alpha$), interlcukin-6 (IL-6), interleukin-8 (IL-8), vascular endothelial growth factor (VEGF), and/or riboflavin carrier protein (RCP) are associated with breast cancer. Of these, RCP, TNF-$\alpha$, IL-8, and VEGF are more informative as to breast cancer status than the other two markers.

Human biochemistry is a complex system wherein many components serve multiple regulatory and other functions and are regulated by multiple other components. Often, biological data are non-Gaussian, particularly in a disease stale. As such, it is common to find biomarkers that display non-Gaussian distributions where measured values can include values that lie substantially apart from the bulk of the values, at the far high end, far low end, or both the high and low end of the distribution, and may span several orders of magnitude. The biomarker analysis system 100 may process the data to accommodate effects of nun-Gaussian distributions. Unlike Gaussian distributions, non-Gaussian distributions may be skewed to the left or to the right with respect to a data mean. Non-Gaussian distributions can be mathematically transformed to Gaussian distributions using logarithmic transformation. Non-Gaussian data can be subjected to sub-group averaging, data segmenting, using differential distributions, or using non-parametric statistics.

To integrate a plurality of biomarkers and control any adverse impact of non-Gaussian data points on the disease status model, the biomarker analysis system may pre-process the biomarker data to generate additional data to facilitate the analysis. For example, the biomarker analysis system 100 may impose various constraints upon, make adjustments to and/or calculate additional data from the raw biomarker level data to generate supplemental data comprising a set of variables in addition to the raw data that may be processed, for example using logistic regression to generate a linear model or other appropriate Statistical analysis that describes the relationship of the biomarkers to the disease state.

For example, the biomarker analysis system 100 may be configured to process the raw biomarker data to reduce negative effects of non-Gaussian distributions. In one embodiment, the biomarker analysis system 100 may reduce the influence of non-normal biomarker levels in biomarkers with non-Gaussian distributions, such as by assigning maximum and/or minimum allowable values or caps for each such biomarker. The caps may be assigned according to any suitable criteria, such as to encompass between about 66% and about 99.7% of the measured levels and exclude extraordinarily high values.

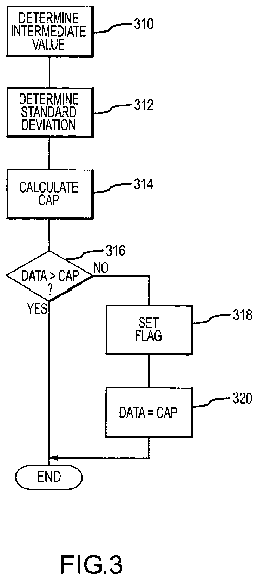

Referring to Figure 3, the maximum and/or minimum allowable values for each candidate biomarker may be established by first determining an intermediate value (310), such as the mean or median value, of that biomarker in the disease-negative cohort, and determining the standard deviation of n selected quantity of the measured biomarker levels (312), such as approximately 30% - 45% of the data points on either side of the median value when the data is plotted on a histogram, such that the central 60% to 90% of the measured data points are accounted for in determining the standard deviation. A maximum allowable value may be determined (314) according to the intermediate value and the standard deviation of the selected biomarker data, for example by adding to the median value to a multiple of the standard deviation, such as no more than four times the standard deviation, and more typically, an amount between one and a half and three times the standard deviation.

In the present embodiment, the biomarker analysis system 100 uses the median, instead of the mean, as the basis for determining the allowed maximum to more accurately reflect the majority of the values while reducing the impact of one or a few very high outlying, non-Gaussian values. Maximum values may also be calculated using data from any suitable set of data and any suitable technique or algorithm, such as data from a disease-positive cohort or from a mixture of disease-positive and disease-negative subjects. Maximum values may be calculated for each of the relevant biomarkers.

For example, in an embodiment of the present invention configured for detecting the presence of breast cancer, the maximum values for the applicable biomarkers are calculated by adding the median value of the biomarker for all subjects without breast cancer to two-and-a-half times the standard deviation of the marker for all subjects without breast cancer. In this exemplary embodiment, suitable median values for PSA, IL-6, TNF-$\alpha$. IL-8, and VEGF may be within ranges of 0.01 -10, 0.5-25, 0.1 -10, 5-150, and 100-5,000 picograms per milliliter (pg/ml) respectively, such as .53, .34, 2.51, 52.12, and 329.98 pg/ml, respectively. Maximum values may he assigned for each of the biomarkers PSA, IL-6, TNF-$\alpha$, IL-8, and VEGF, for example within the ranges of 5-200, 10-300, 0.5-50, 100-2,000, and 500- 10,000 pg/ml, respectively, such as 122.15, 12.52, 48.01, 350.89, and 821.15 pg/ml, respectively. Thus, different maximum values may be calculated for the PSA, IL-6, RCP, TNF-$\alpha$, IL-8, and VEGF biomarkers, or for the RCP, TNF-$\alpha$, IL-8, and VEGF biomarkers alone. In the present embodiment, these figures are determined using ELISA measurements for healthy women. The values may change as more data is added, variations in the ELISA procedure and/or test kits, reliance on data for disease-positive women, or use of non-ELISA techniques.

The resulting maximum allowable value may then be compared to the individual measured biomarker levels (316). If a particular subject's measured level is above the maximum value, a modification designator or flag, such as an integral value of 1 or 0 or other appropriate designator, may be associated with the subject's biomarker data, such as recorded in a particular field in his or her supplemental data set; if the biomarker level is below the maximum, an integral value of 0 is recorded in his or her supplemental data set (318). The designator criteria may be applied consistently between generating a disease status model and scoring an individual patient's biomarker levels to ease disease status model interpretation. The designators may also comprise more than just two discrete levels.

Additionally, when any of a subject's biomarker values exceed the maximum allowable value for that biomarker, the raw biomarker values may be replaced with the maximum allowable value for that biomarker (320). The adjusted data having capped values and additional designators may be part of the supplemental data, so that the raw data is preserved and the adjusted data with capped values and additional designators become part of the supplemental data set. The additional designator denotes that the measured values were unusually high, which may be informative about the disease status, while the replacement with the cap value limits the influence of the extremely high values. Without such caps, the extremely high values may "pull" the linear model to fit data that is the exception, not the norm.

Thus, if the patient's RCP biomarker exceeds the maximum allowable value, a flag is set in the subject's supplemental data to indicate that the RCP biomarker exceeded the limit and the raw biomarker level may be replaced with the maximum allowable value. Conversely, if the TNF-$\alpha$ biomarker level is within the range of accepted values, the original biomarker level is retained and the corresponding flag in the subject's supplemental data remains unset.

The biomarker analysis system 100 may also be configured to generate and analyze normalized data, for example based on the raw biomarker data and/or the capped supplemental data. Normalized data comprises the original data adjusted to account for variations observed in the measured values that may be attributed to one or more statistically significant biomarker level-affecting factors. For example, genetic, behavioral, age, medications, or other factors can increase or decrease the observed levels of specific biomarkers in an individual, independent of the presence or absence of a disease state. In the present embodiment, to detect breast cancer, potential factors that may substantially affect the levels of biomarkers indicative of breast cancer include: age; menopausal status; whether a hysterectomy has been performed; the usage of various hormones such birth control, estrogen replacement therapy. Tamoxifen or Raloxifene, and fertility drugs; the number or full-term pregnancies; the total number of months engaged in breast-feeding; prior breast biopsies; prior breast surgeries; a family history of breast cancer; height; weight; ethnicity; dietary habits; medicinal usage, including the use of NSAIDs; presence of other diseases; alcohol consumption; level of physical activity; and tobacco use.

Any suitable source or system may be used to identify factors that may affect a given biomarker, such as literature and research. In addition, any suitable processes or techniques may be used to determine whether particular factors are applicable and to what degree. For example, upon collecting the biological samples, members of the cohorts can be queried through subject questionnaires, additional clinical tests, or other suitable processes and mechanisms about various factors that can possibly affect the levels of their markers. The subject data containing this information relating to the subjects themselves may he provided to the biomarker analysis system 100 with the raw biomarker data, for example in the form of discrete and/or continuous variables.

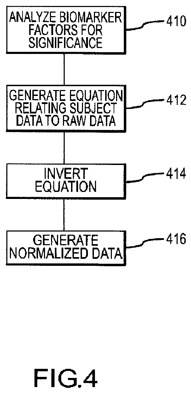

The relevance and effects of various factors upon biomarker levels may be assessed in any suitable manner. For example, when sample collection is completed, all biomarkers have been measured, and the raw data and subject data relating to the additional factors has been provided, the biomarker analysis system 100 may analyze the raw data and additional factors to identify such factors with a statistically significant affect. The biomarker analysis system 100 may also automatically select multiple relevant biomarkers from the plurality of biomarkers. In one embodiment, referring to Figure 4, the biomarker analysis system 100 performs regression analyses or other appropriate statistical analyses using each biomarker as a dependent variable and the factors that potentially affect its level as independent variables (410). The biomarker analysis system 100 may, however, use any appropriate analysis to identify potential relationships between the factors and variations in the biomarker data.

In the present embodiment, factors that are found to retain a p-value below a predetermined level (e.g., without limitation, $p < 0.1$, $p < 0.05$, or $p < 0.025$) may be considered significant. The biomarker analysis system 100 may also be configured to compensate for the effects of such factors, such as by generating normalized data wherein the variation attributable to such factors has been removed from the analysis. For example, to remove factor-ascribed variation, raw data may be transformed using the inverse of a linear equation describing the relationship between the biomarker level and the factor or factors found to be significant. In one example of the present invention, the selected p-value to determine statistical significance for biomarkers specific to detecting breast cancer may be selected at .05. In another particular example, should linear regression or other appropriate analysis of raw data and subject show that a subject's age and gender affect a potential biomarker relating to Alzheimer's disease Y to a statistically significant level, the relationship the observed biomarker levels and the subject's age and gender could be described by the equation:

$$Y = M_1(Age) + M_2(Male) + B$$

where Y is the measured level of the potential Alzheimer's disease biomarker, $M_1$ and $M_2$ are the coefficients as determined by the linear regression, (Age) is a continuous variable that was found to be a statistically significant determinate of Y, (Male) is a discrete variable that was found to be a statistically significant determinate of Y, where 1 equals male and 0 equals female, and B is an intercept (412). To remove the variation in Y that can be ascribed to age and gender, a normalized or adjusted value Y' for the potential Alzheimer's disease biomarker Y may be calculated according to the inverse equation (414):

$$Y' = Y*(1/M_1)(Age) - M_2(Male)$$

Normalized data may be generated applying the inverse equation to the raw data and/or the supplemental data and added to the supplemental data. By removing variation due to known causes, a greater percentage of the remaining variation may be ascribed to the presence or absence of a disease state, thus clarifying a marker's relationship to the disease state that might otherwise be obscured. When statistically significant factors are identified as affecting the level of one or more potential biomarkers, both raw data and normalized data may he used in subsequent analyses. Analysis of normalized values may elucidate relationships that would otherwise be obscured, while raw data may provide greater ease of test administration and delivery.

The biomarker analysis system 100 may further process the raw and/or supplemental data in any suitable manner, such as to reduce the influence of non-Gaussian distributions. For example, the biomarker analysis system 100 may select one or more biomarker cut points and compare the raw and/or supplemental biomarker data to at least one designated biomarker cut point. Biomarker cut points may be selected according to any suitable criteria, such as according to known levels corresponding to disease or based on the raw and/or normalized biomarker data. For example, the biomarker analysis system 100 may compare cumulative frequency distributions of the condition-positive and -negative biomarker data for a particular biomarker and select one or more cut points for the biomarker according to a maximum difference between the condition-positive cumulative frequency distribution and the condition-negative cumulative frequency distribution for the selected biomarker.

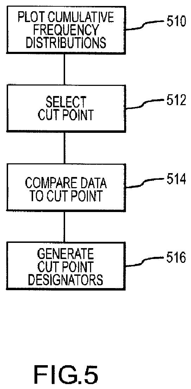

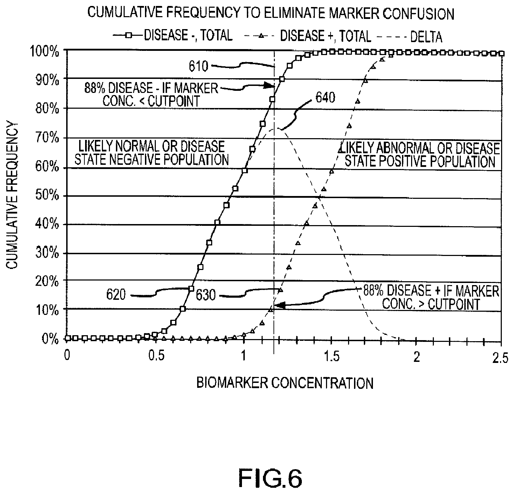

In one embodiment, referring to Figures 5 and 6, the biomarker analysis system 100 designates at least one cut point for each biomarker. The biomarker analysis system 100 may initially generate cumulative frequency distributions for the raw and/or supplemental data for both the disease-positive cohort 630 and the disease-negative cohort 620 for each relevant biomarker (510), such as for each individual biomarker PSA, IL-6, RCP, TNF-$\alpha$, IL-8, and VEGF. The biomarker analysis system 100 may select one or more cut points (512), for example at a level where the difference between the cumulative frequency distribution of measured values in the disease-positive cohort and in the disease-negative cohort exceeds a predetermined value. The predetermined value may be any suitable threshold, such as where the cumulative frequency difference exceeds 10%, with higher values indicating greater difference between the positive and negative cohorts.

The present biomarker analysis system 100 may seek levels at which the difference between the positive and negative cohorts is greatest to establish cut points 640. A greater difference in the cumulative frequencies of the disease-positive and -negative states indicates a propensity to belong to either the disease-positive or disease-negative cohort. Conversely, potential markers that display less than a 10% difference in cumulative frequency at any point are less likely to be informative to a useful extent and may optionally be dropped from further analysis.

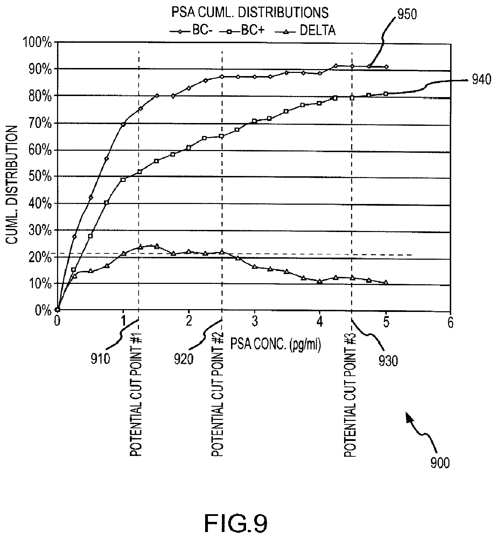

A cut point 640 may be selected even where the differences in cumulative frequency are low, particularly where the cut point may be deemed to be particularly informative, such as in the ease where there are no disease-positive or disease-negative values beyond a certain biomarker level. For example, referring to Figure 9, to detect breast cancer, cut-points for the biomarker PSA may be selected for values that are at a local maximum with an absolute difference exceeding 10% using a cumulative frequency plot 900. In this embodiment, a first cut point 910 is selected at 1.25, a second cut point 920 is selected at 2.5, and a third cut point 930 is selected at 4.5. The differences in the cumulative frequency between disease-positive cohort plot 940 and disease-negative cohort plot 950 at each of the three cut points are 24%, 22%, and 12% respectively. In this embodiment, the third cut point 930 may be suitably selected despite the relatively low difference in cumulative frequency since the lack of disease-negative values beyond a PSA concentration of 4.5 indicates a point that is particularly informative to the distribution.

Referring again to Figure 5, the raw and/or normalized biomarker data may he compared to the cut points (514) and the biomarker analysis system 100 may record a value indicating the result of the comparison as a cut point designator (516). The cut point designator may comprise any suitable value or indicator, such as the difference between the value and the cut point or other value. In one embodiment, if a raw or normalized biomarker level is above the cut point, un integral value of 1 is recorded as the cut point designator and stored in the supplemental data; if the level is below the cut point, an integral value of 0 is recorded. The integral values could likewise indicate whether the biomarker levels are below the more than one cut-point, or exceed a cut point for some of a patient's biomarkers and not exceeding a cut point for others. Conversion of a continuous variable into a discrete variable indicates a propensity to belong to either a disease-positive or -negative cohort. All values on a particular side of a cut point may receive equal weight, regardless of how high or low they may be, which tends to eliminate the influence of non-Gaussian distributions.

The biomarker analysis system 100 may also be configured to reduce the range of values in data, for example where the range of measured or normalized level values for a biomarker is extremely wide. The range of values may be narrowed and the number of extremely high values reduced, while maintaining a meaningful distinction between values at the low and high ends of the range. The biomarker analysis system 100 may adjust the range of values in any suitable manner, for example by raising the measured values to fractional powers to obtain a set of reduced values for the biomarker. The biomarker analysis system 100 may select any suitable exponent values to maintain meaningful distinctions in the data. Meaningful distinctions can be lost if the range is narrowed too much by choosing a fractional power that is too small.

In the present embodiment, the biomarker analysis system may adjust the measured value for each biomarker, such as the PSA, IL-6, RCP, TNF-$\alpha$, IL-8, and VEGF biomarkers, in each cohort member by raising each value to a fractional power. Multiple different fractional powers, such as exponential values ranging from ¾ to 1/10, such as 2/3 and 1/2, can be included in the analysis for each biomarker. Each reduced value may be Included in the supplemental data associated with the relevant biomarker's data set. The biomarker analysis system 100 may analyze the results, such as in the course of performing later regression analysis, to identity the fractional power value(s) that best accommodates the data, for example by removing those sets of values that lack statistical significance. Exponentially raising measured or normalized level values by fractional values reduces the data's range, allows linear models to better fit non-linear data, and provides a continuum of scoring where differing weights can be applied as high or low values. In an embodiment configured to detect breast cancer, for example, suitable fractional powers for the PSA, IL-6, RCP, TNF-$\alpha$, IL-8, and VEGF biomarkers may include 1/10, 1/5, 1/3, 1/2, and 2/3 for each of the relevant biomarkers.

The biomarker analysis system 100 may generate the disease status model on the raw data, the normalized data, any other supplemental data, and/or any additional disease risk factors that may have an impact or influence on specific risk for development of a disease. Given the complexity of human biochemistry, many factors can affect the measured concentration of one or more biomarkers, including, but not limited to, a patient's demographic characteristics, family history, and medical history. These factors all increase the potential markers' observed variabilities and standard deviations, masking or obscuring the relationship to the disease state.

The biomarker analysis system 100 may analyze and/or process disease risk factors that can affect a subject's risk, as well as biomarker factors that can affect biomarker levels differently as described above. The biomarker analysis system 100 may, for example, account for disease risk factors in the overall analysis of the data in conjunction with analyzing the marker specific scores. Considering risk factors accounts for differences in prevalence and essentially shifts the overall score to reflect the prevalence.

For example, as with the biomarker factors that can influence measured biomarker levels, disease risk factors may be included among the identified variables in determining the relationship between the variables and disease status. The additional disease risk factors may be selected according to any suitable criteria and/or from any suitable source. For example, technical literature may identify additional factors that have an impact or influence on specific risk for development of a particular disease of interest. Specific risk factors may include, without limitation, age, race, family history, date of menarche, menopausal status, depression, disease status, medication status, body mass index (BMI), date of first childbirth, head injuries, and/or other factors. When such disease risk factors arc known or suspected to be associated with a disease state, the subject's medical histories and/or the actual subject should be queried about the disease risk factors. This additional subject data may be provided to the biomarker analysis system 100, which may record the subjects' disease risk factor data with the subjects' biomarker (actor data as additional continuous or discrete variables.

The biomarker analysis system 100 suitably analyzes the data to identify relationships between the disease state and various raw data, supplemental data, and/or subject data. The relationship may be identified according to any suitable analysis and criteria. For example, the biomarker analysis system 100 may establish an equation, such as a linear equation, that describes a relationship between the identified variables and disease status. The biomarker analysis system 100 may apply any suitable analysis, such us one or more conventional regression analyses (e.g., linear regression, logistic regression, and/or Poisson regression) using the disease status as the dependent variable and one or more elements of the raw data and the supplemental data as the independent variables, or employ other analytical approaches, such as a generalized linear model approach, logit approach, discriminant function analysis, analysis of covariance, matrix algebra and calculus, and/or receiver operating characteristic approach. In one embodiment, the biomarker analysis system 100 automatically generates a statistical model for determining disease status according to differences between the biomarker data for the relevant biomarkers of the respective cohorts.

The present biomarker analysis system 100 may assess the relevance of a biomarker to a particular disease or condition according to any suitable technique or process. In one embodiment, the biomarker analysis system 100 performs statistical analyses of the biomarker data, such as statistical significance analyses. For example, the biomarker analysis system 100 may automatically generate a disease status model that eliminates non-informative and some less informative biomarkers, for example by disregarding all potential biomarkers that yield p-values less than a predetermined value upon statistical analysis against the disease status. The biomarker analysis system 100 may determine the relative contribution or strength of the remaining individual biomarkers, for example by the coefficients that the model applies to the markers or by the product of the coefficient of each marker and its range of values. Higher coefficients or products relative to those for other biomarkers in the model indicate more impact that the biomarker may be assigned for determining the disease state in the disease status model. In the present embodiment, the analysis may reduce the number of cut points and fractional exponent values used, in many cases to a single cut point and/or fractional exponent. Some of the factors are likely to relate to duplicate Information, so the biomarker analysis system 100 may select the factor that is most useful, such as the factor having the lowest p-value.

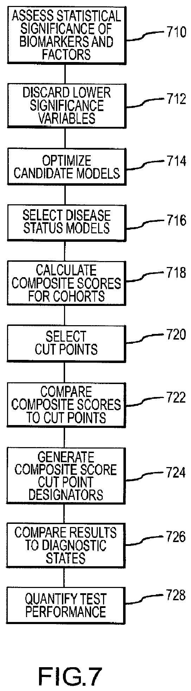

Referring to Figure 7, the biomarker analysis system 100 may perform an iterative analysis either starting with a single variable and adding variables one at a time, or starting with all variables and removing variables one at a time, until all variables are determined to be statistically significant, such as by having p-values lower than a predetermined level (e.g., without limitation, $p<0.1$, $p<0.05$, or $p<0.025$) (710). The iterative analysis may be configured to identify and remove biomarker data that is less informative regarding disease status than other data. For example, independent variables that demonstrate a p-value less than a predetermined value are retained in the model, while those with p-values higher than the predetermined value are discarded (712). The biomarker analysis system 100 may analyze multiple variations of additions and subtractions of variables to acquire an optimal solution (714), for example to maximize the model's adjusted R squared or the Bayesian information criterion and avoid sub-optimizing the model. For example, the resultant scoring model may take the form of the following equation:

$$y = m_1x_1 + m_2x_2 + m_3x_n + m_4d_1 + m_5d_2 + m_5d_n + b$$

where y is a continuous variable representing disease status;

$X_{l-n}$ are continuous variables, such as raw biomarker levels measured in biological samples and/or normalized or capped values which have been identified as statistically significant, such as raw and supplemental data for the RCP, TNF-$\alpha$, IL-8, and VEGF biomarkers;

$d_{l-n}$ are the discrete variables, such as discrete disease risk factors or designators in the supplemental data, that have been identified as statistically significant,

$m_l$ $m_n$ are coefficients associated with each identified variable, and

b is the y-intercept of the equation.

When the remaining variables are defined and their respective coefficients are selected, the biomarker analysis system 100 establishes the resulting equation as the disease status model (716). The biomarker analysis system 100 may establish multiple disease status models as candidates for further evaluation. The biomarker analysis system 100 may generate composite scores for various subjects in the relevant cohorts by multiplying values for the variables in the disease status model by the coefficient determined during modeling and adding the products along with the intercept value (718). The disease status model may comprise, however, any suitable model or relationship for predicting disease status according to the raw data, supplemental data, and/or subject data.

The biomarker analysis system 100 may utilize the results of the analysis of relationships between the disease state and various raw data, supplemental data, and/or subject data to establish diagnosis criteria for determining disease status using data identified as informative. The biomarker analysis system 100 may establish the diagnosis criteria according to any appropriate process and/or techniques. For example, the biomarker analysis system 100 may identify and/or quantify differences between informative data (and/or combinations of informative data) for the disease-positive cohort, and corresponding informative data (and/or combinations of informative data) for the disease-negative cohort.

In the present embodiment, the biomarker analysis system 100 compares the composite scores for the respective cohorts to identify one or more cut points in the composite that may indicate a disease-positive or -negative status, for example, the biomarker analysis system 100 may select and/or retrieve one or more diagnosis cut points and compare the composite scores for the respective cohorts to the diagnosis cut points (722). The diagnosis cut points may be selected according to any suitable criteria, such as according to differences in median and/or cumulative frequency of the composite scores for the respective cohorts. Alternatively, the cut points may be regular intervals across the range of composite scores.

The biomarker analysis system 100 may compare the composite score for each member of a cohort to one or more cut points and record a value indicating the result of the comparison as a composite score cut point designator (724). The composite score cut point designator may comprise any suitable value or indicator, such as the difference between the value and the cut point or other value. In one embodiment, If a composite score is above the cut point, an integral value of 1 is recorded as the composite score cut point designator; if the level is below the cut point, an integral value of 0 is recorded. The integral values could likewise indicate whether the composite scores are below more than one cut point.

In the present embodiment, to determine the appropriate cut-point for determining disease-positive or disease-negative status, each cohort subject's composite score is suitably evaluated at different cut-points which span the data's range. At each cut point, values that are equal to or less than the cut point may be considered disease-negative and values above the cut point may be considered disease-positive point, or vice versa according to the nature of the relationship between the data and the disease. The biomarker analysis system 100 may compare the composite score cut point designator for each cut point candidate to each cohort member's true diagnostic state (726), and quantify the test's performance at each cut-point (728), for example as defined by sensitivity, specificity, true positive fraction, true negative Traction, false positive fraction, false negative traction, and so on. From the range of evaluated cut-points, the biomarker analysis system 100 may select one or more cut points for future evaluations of data such that sensitivity is maximized specificity is maximized, or the overall lest performance is maximized as a compromise between maximum sensitivity and specificity.

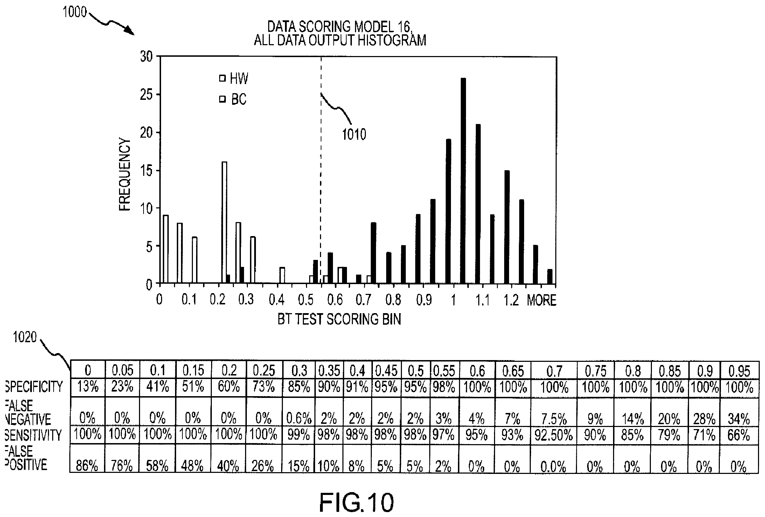

In an exemplary embodiment of the present invention configured to detect the presence of breast cancer, referring now to Figure 10, an appropriate cut point may be selected by using a data scoring model 1000. In this embodiment, the data scoring model 1000 includes a table 1020 that indicates lest accuracy for specificity and sensitivity at various cut points. Using the data provided in the table 1020, the biomarker analysis system 100 may select a cut point 1010 to provide an optimum balance between sensitivity and specificity, such as at .55 in the present exemplary embodiment.

The biomarker analysis system 100 may also be configured to verify validity of the disease status model. For example, the biomarker analysis system 100 may receive blind data from disease-negative and disease-positive individuals. The blind data may be analyzed to arrive at diagnoses that may he compared to actual diagnoses to confirm that the disease stale model distinguishes disease-negative and disease-positive solely on the basis of the values of measured and determined variables. If several models are viable, the model that has the highest agreement with the clinical diagnosis may be selected for further evaluation of subjects.

After the disease status model has been established, the biomarker analysis system 100 may analyze biological sample data and/or subject data to apply the disease status model as an indicator of disease status of individual patients. The relevant biomarker levels may be measured and provided to the biomarker analysis system 100, along with relevant subject data.

The biomarker analysis system 100 may process the biomarker data and subject data, for example to adjust the biomarker levels in view of any relevant biomarker factors. The biomarker analysis system 100 may not utilize various variables, such as one or more integral values associated with a biomarker specific cut-point, reduced values, integral values denoting extraordinary values, and raw or normalized data. Data that is not needed for the particular disease status model may be discarded. The biomarker analysis system 100 may use and/or generate only relevant biomarkers and variables, which are those that demonstrate statistical significance and/or are used in the disease status model, to evaluate individual patients. For example, if the disease status model originally considered the PSA, TL-6, RCP, TNF-$\alpha$, IL-8, and VEGF biomarkers, but discarded the PSA and IL-6 biomarkers as insignificant or less significant biomarkers, the biomarker analysis system 100 may discard data for the PSA and IL-6 biomarkers and proceed with analysis of the RCP, TNF-$\alpha$, IL-8, and VEGF biomarkers.

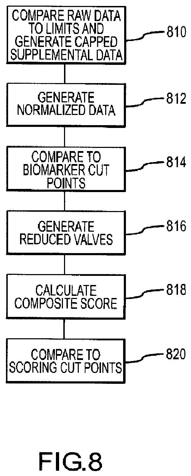

Referring to Figure 8, the biomarker analysis system 100 may perform any suitable processing of the raw biomarker data and other patient information. For example, the biomarker analysis system 100 may establish for each of the patient's relevant biomarker levels a designator, such as an integral value, that indicates whether the level for each biomarker exceeds the relevant biomarker-specific maximum allowable value designated in the disease status model (810). The biomarker analysis system 100 may also associate the corresponding designators with the patient's supplemental data set, indicating that the raw value exceeded the relevant limit.

In addition, the biomarker analysis system 100 may generate normalized data for the patient according to the normalization criteria established in generating the disease status model and the subject data for the patient, such as the patient's age, smoking habits, and the like (812). The normalized data may be added to the supplemental data for the patient.

The biomarker analysis system 100 may also compare the patient's raw data and/or supplemental data to the biomarker cut points and generate cut point designators for each relevant biomarker cut point and the corresponding data (814). The biomarker analysis system 100 may further establish reduced data values for the each of the patient's relevant measured biomarker levels, for example by raising the relevant data to the fractional powers used by the disease status model, and associating alt such reduced data values with the patient's data set (816).

The biomarker analysis system 100 may evaluate the raw biomarker data and any other relevant data in conjunction with the disease status model. For example, the biomarker analysis system 100 may calculate a composite score for the patient using the patient's biomarker data and other data and the disease status model (818). The biomarker analysis system 100 may compare the composite score to the scoring model cut points (820). Scores above the cut point suggest that the disease status of the subject is positive, while scores below the cut point indicate that the subject is negative. The biomarker analysis system 100 may also compare the composite score to boundary definitions for indeterminate zone that may be constructed around the cut-point where no determination can be made. The indeterminate zone may account, for example, for both a patient's biological variability (the typical day to day variations in the biomarkers of interest) and the evaluation methods error.

The particular implementations shown and described are illustrative of the invention and its best mode and are not intended to otherwise limit the scope of the present invention in any way. Indeed, for the sake of brevity, conventional processing, data entry, computer systems, and other functional aspects of the system may not be described in detail. Furthermore, the connecting lines shown in the various figures are intended to represent exemplary functional relationships and/or physical couplings between the various elements. Many alternative or additional functional relationships or physical connections may be present in a practical system.

The present invention has been described above with reference to a particular embodiment. However, changes and modifications may be made to the particular embodiment without departing from the scope of the present invention. These and other changes or modifications are intended to be included within the scope of the present invention. There have been disclosed hereinbefore methods, systems and computer programs defined by the following numbered paragraphs.

1. A method for assessing a disease status of a human, comprising:

obtaining condition-positive biomarker data for a plurality of biomarkers for a condition-positive cohort;
obtaining condition-negative biomarker data for the plurality of biomarkers for a condition-negative cohort;
automatically selecting multiple relevant biomarkers from the plurality of biomarkers; and
generating a statistical model for determining the disease status according to a difference between the biomarker data for the relevant biomarkers of the condition positive cohort and the biomarker data for the relevant biomarkers of the condition negative cohort.

2. The method of paragraph 1, further comprising:

generating a reduced range biomarker data for the relevant biomarkers of the condition-positive cohort and the reduced range biomarker data for the relevant biomarkers of the condition-negative cohort; and
wherein generating the statistical model includes generating the statistical model according to:

the difference between the biomarker data for the relevant biomarkers of the condition-positive cohort and the biomarker data for the relevant biomarkers of the condition-negative cohort; and
a difference between the reduced range biomarker data for the relevant biomarkers of the condition-positive cohort and the reduced range biomarker data for the relevant biomarkers of the condition-negative cohort.

3. The method of paragraph 2, wherein generating the reduced range biomarker data comprises multiplying the biomarker data by a fractional exponent.

4. The method of paragraph 1, further comprising:

comparing a cumulative frequency distribution of the condition-positive biomarker data tor a selected biomarker to a cumulative frequency distribution of the condition-negative biomarker data for the selected biomarker;
selecting a cut point for the biomarker according to a maximum difference between the condition-positive cumulative frequency distribution and the condition negative cumulative frequency distribution for the selected biomarker.

5. The method of paragraph 4, farther comprising:

comparing the condition-positive biomarker data and the condition-negative biomarker data to the cut point; and
generating a cut point data set comprising a set of discrete values according to whether each datum compared to the cut point exceeded the cut point.

6. The method of paragraph 1, wherein generating a statistical model comprises performing an iterative analysis on the biomarker data for the relevant biomarkers of the condition positive cohort and the biomarker data for the relevant biomarkers of the condition negative cohort, wherein the iterative analysis is configured to identify and remove a first set of data that is less informative as to disease status than a second set of data.

7. The method of paragraph 1, further comprising:

comparing the condition-positive biomarker data and the condition-negative biomarker data to a threshold; and generating multiple discrete values for the condition-positive biomarker data and the condition-negative biomarker data compared to the threshold according to a result of the comparison; and generating the statistical model for determining the disease status according to differences between the discrete values for the relevant biomarkers of the condition positive cohort and the discrete values tor the biomarker data for the relevant biomarkers of the condition-negative cohort.

8. The method of paragraph 7, further comprising generating capped biomarker data for the condition-positive biomarker data and the condition-negative biomarker data, wherein capped biomarker data comprises:

the condition-positive biomarker data and the condition-negative biomarker data for data within a cap limit; and a cap value for condition-positive biomarker data and the condition-negative biomarker data that exceeds the cap limit.

9. The method of paragraph 8, further comprising selecting the cap limit according to a median value of al least one of the condition-positive biomarker data and the condition-negative biomarker data.

10. The method of paragraph 1, wherein:

the Statistical model includes at least one dependent variable and more than one independent variable, the disease status is a dependent variable; and a plurality of magnitudes for the multiple relevant biomarkers comprise a plurality of independent variables.

11. A system for assessing a disease status of a human, comprising a computer system configured to:

receive condition-positive biomarker data for a plurality of biomarkers for a condition-positive cohort; receive condition-negative biomarker data for the plurality of biomarkers for a condition-negative cohort; automatically select multiple relevant biomarkers from the plurality of biomarkers; and automatically generate a statistical model for determining the disease status according to a difference between the biomarker data for the relevant biomarkers of the condition-positive cohort and the biomarker data for the relevant biomarkers of the condition-negative cohort.

12. The system of paragraph 11, wherein the computer system is further configured to:

generating a reduced range biomarker data for the relevant biomarkers of the condition-positive cohort and the reduced range biomarker data for the relevant biomarkers of the condition-negative cohort; and automatically generate the statistical model according to:

the difference between the biomarker data for the relevant biomarkers of the condition-positive cohort and the biomarker data for the relevant biomarkers of the condition-negative cohort; and a difference between the reduced range biomarker data for the relevant biomarkers of the condition-positive cohort and the reduced range biomarker data for the relevant biomarkers of the condition-negative cohort.

13. The system of paragraph 12, wherein the computer system is configured generate the reduced range biomarker data by multiplying the biomarker data by a fractional exponent.

14. The system of paragraph 11, wherein the computer system is further configured to:

compare a cumulative frequency distribution of the condition-positive biomarker data for a selected biomarker to a cumulative frequency distribution of the condition negative biomarker data for the selected biomarker; select a cut point for the biomarker according to a maximum difference between the condition-positive cumulative frequency distribution and the condition-negative cumulative frequency distribution for the selected biomarker.

15. The system of paragraph 14, wherein the computer system is further configured:

compare the condition-positive biomarker data and the condition-negative biomarker data to the cut point; and generate a cut point data set comprising a set of discrete values according to whether each datum compared to the cut point exceeded the cut point.

16. The system of paragraph 11, wherein the computer system is configured to automatically generate a statistical model by performing an iterative analysis on the biomarker data for the relevant biomarkers of the condition-positive cohort and the biomarker data for the relevant biomarkers of the condition-negative cohort, wherein the iterative analysis is configured to identify and remove a first set of data that is less informative as to disease status than a second set of data.

17. The system of paragraph 11, wherein the computer system is further configured to:

compare the condition-positive biomarker data and the condition-negative biomarker data to a threshold; and generate multiple discrete values for the condition-positive biomarker data and the condition-negative biomarker data compared to the threshold according to a result of the comparison; and automatically generate the statistical model for determining the disease status according to differences between the discrete values for the relevant biomarkers of the condition-positive cohort and the discrete values for the biomarker data for the relevant biomarkers of the condition-negative cohort.

18. The system of paragraph 17, wherein the computer system is further configured generate capped biomarker data for the condition-positive biomarker data and the condition negative biomarker data, wherein capped biomarker data comprises:

the condition-positive biomarker data and the condition-negative biomarker data for data within a cap limit; and a cap value for condition-positive biomarker data and the condition-negative biomarker data that exceeds the cap limit.

19. The system of paragraph 18, wherein the computer system is further configured select the cap limit according to a median value of at least one of the condition-positive biomarker data and the condition-negative biomarker data.

20. The system of paragraph 11, wherein:

the statistical model includes at least one dependent variable and more than one independent variable, the disea.se status is a dependent variable; and a plurality of magnitudes for the multiple relevant biomarkers comprise a plurality of independent variables.

21. A computer program configured to cause a computer to execute a method for assessing a disease status of a human, the method comprising:

obtaining condition-positive biomarker data for a plurality of biomarkers for a condition-positive cohort; obtaining condition-negative biomarker data for the plurality of biomarkers for a condition-negative cohort; automatically selecting multiple relevant biomarkers from the plurality of biomarkers; and automatically generating a statistical model for determining the disease status according to a difference between the biomarker data for the relevant biomarkers of the condition-positive cohort and the biomarker data for the relevant biomarkers of the condition-negative cohort.

22. The computer program of paragraph 21, the method further comprising generating a reduced range biomarker data for the relevant biomarkers of the condition-positive cohort and the reduced range biomarker data tor the relevant biomarkers of the condition-negative cohort; and wherein automatically generating the statistical model includes generating the statistical model according to:

the difference between the biomarker data for the relevant biomarkers of the condition-positive cohort and the biomarker data for the relevant biomarkers of the condition-negative cohort; and

a difference between the reduced range biomarker data for the relevant biomarkers of the condition-positive cohort and the reduced range biomarker data for the relevant biomarkers of the condition-negative cohort.

23. The computer program of paragraph 22, wherein generating the reduced range biomarker data comprises multiplying the biomarker data by a fractional exponent.

24. The computer program of paragraph 21, the method further comprising:

comparing a cumulative frequency distribution of the condition-positive biomarker data for a selected biomarker to a cumulative frequency distribution of the condition-negative biomarker data for the selected biomarker; selecting a cut point for the biomarker according to a maximum difference between the condition-positive cumulative frequency distribution and the condition negative cumulative frequency distribution for the selected biomarker.

25. The computer program of paragraph 24, the method further comprising:

comparing the condition-positive biomarker data and the condition-negative biomarker data to the cut point; and generating a cut point data set comprising a set of discrete values according to whether each datum compared to the cut point exceeded the cut point.

26. The computer program of paragraph 21, wherein automatically generating a statistical model comprises, performing an iterative analysis on the biomarker data for the relevant biomarkers of the condition-positive cohort and the biomarker data for the relevant biomarkers of the condition-negative cohort, wherein the iterative analysis is configured to identify and remove a first set of data that is less informative as to disease status than a second set of data.

27. The computer program of paragraph 21, the method further comprising:

comparing the condition-positive biomarker data and the condition-negative biomarker data to a threshold; and generating multiple discrete values for the condition-positive biomarker data and the condition-negative biomarker data compared to the threshold according to a result of the comparison; and automatically generating the statistical model for determining the disease status according to differences between the discrete values for the relevant biomarkers of the condition-positive cohort and the discrete values for the biomarker data for the relevant biomarkers of the condition-negative cohort.

28. The computer program of paragraph 27, the method further comprising generating capped biomarker data for the condition-positive biomarker data and the condition-negative biomarker data, wherein capped biomarker data comprises:

the condition-positive biomarker data and the condition-negative biomarker data for data within a cap limit; and a cap value for condition-positive biomarker data and the condition-negative biomarker data that exceeds the cap limit.

29. The computer program of paragraph 28, the method further comprising selecting the cap limit according to a median value of at least one of the condition-positive biomarker data and the condition-negative biomarker data.

30. The computer program of paragraph 21, wherein:

the statistical model includes at least one dependent variable and more than one independent variable, the disease status is a dependent variable; and a plurality of magnitudes for the multiple relevant biomarkers comprise a plurality of independent variables.

**Claims**

1.  A method for assessing a breast cancer disease status of a patient, comprising:

    obtaining a first data set for a plurality of biomarkers in a condition-positive cohort;
    obtaining a second data set for a plurality of biomarkers in a condition-negative cohort;
    calculating a composite score for each member of the condition-positive cohort and the condition negative cohort by multiplying values for biomarker variables by a coefficient, adding the resulting products together and adding an intercept value;
    determining a median value of the composite score for each member of the condition-positive cohort and condition-negative cohort;
    calculating a cutpoint value related to the median value, wherein the cutpoint value indicates a disease positive or disease negative status;
    generating a disease status model based on the the composite scores and the cutpoint value;
    inputting a patient data set for the at least one of the plurality of biomarkers into the disease status model; and
    determining a disease status of the patient.

2.  The method according to claim 1, further comprising a step of comparing the composite score for each member of the disease positive cohort and disease negative cohort to one or more cut points to thereby generate a cut point designator value.

3.  The method according to claim 2, further comprising:

    a step of comparing the composite score cut point designator to each cohort member's true diagnostic state, to thereby quantify the performance of the disease status model at each cut point.

4.  The method according to claim 3, further comprising:

    receiving a result from the testing the performance of the disease status model;
    adjusting the cutpoint value; and
    regenerating the disease status model.

5.  The method according to claim 4, further comprising increasing a sensitivity of the disease status model.

6.  The method according to claim 1, further comprising:

    determining at least one risk factor for the disease; and
    regenerating the disease status model with a weighted value for the at least one risk factor for the disease.

7.  The method according to claim 1, further comprising:

    running an iterative analysis on the plurality of biomarkers; and
    eliminating at least one insignificant biomarker from the plurality of biomarkers.

8.  The method according to claim 1, further comprising:

    determining a maximum value for the composite score for each member of the condition-positive cohort; and
    eliminating a member with the composite score greater than or equal to the maximum value from the condition positive cohort.

9.  The method according to claim 10, further comprising:

    determining an unique risk factor in the member with the composite score greater than or equal to the maximum value; and
    eliminating each member having the unique risk factor from the condition-positive cohort.

10. The method according to claim 1, further comprising:

determining a maximum value for the composite score for each member of the condition-positive cohort; and assigning a cap value to any composite score above the maximum value.

11. A computer system for assessing a breast cancer disease status of a patient, comprising a computer system configured to cause a computer to execute the method of any of claims 1-10.

12. A computer program configured to cause a computer to execute a method for assessing a breast cancer disease status of a patient according to the method of any of claims 1-10.

FIG.1

FIG.2

DETERMINE INTERMEDIATE VALUE — 310

DETERMINE STANDARD DEVIATION — 312

CALCULATE CAP — 314

DATA > CAP ? — 316

NO

YES

SET FLAG — 318

DATA = CAP — 320

END

FIG.3

ANALYZE BIOMARKER FACTORS FOR SIGNIFICANCE ──410

GENERATE EQUATION RELATING SUBJECT DATA TO RAW DATA ──412

INVERT EQUATION ──414

GENERATE NORMALIZED DATA ──416

# FIG.4

PLOT CUMULATIVE
FREQUENCY
DISTRIBUTIONS — 510

SELECT
CUT POINT — 512

COMPARE DATA
TO CUT POINT — 514

GENERATE
CUT POINT
DESIGNATORS — 516

FIG.5

CUMULATIVE FREQUENCY TO ELIMINATE MARKER CONFUSION

FIG.6

ASSESS STATISTICAL
SIGNIFICANCE OF
BIOMARKERS AND
FACTORS — 710

DISCARD LOWER
SIGNIFICANCE
VARIABLES — 712

OPTIMIZE
CANDIDATE MODELS — 714

SELECT DISEASE
STATUS MODELS — 716

CALCULATE
COMPOSITE SCORES
FOR COHORTS — 718

SELECT
CUT POINTS — 720

COMPARE
COMPOSITE SCORES
TO CUT POINTS — 722

GENERATE
COMPOSITE SCORE
CUT POINT
DESIGNATORS — 724

COMPARE RESULTS
TO DIAGNOSTIC
STATES — 726

QUANTIFY TEST
PERFORMANCE — 728

# FIG.7

COMPARE RAW DATA
TO LIMITS AND
GENERATE CAPPED
SUPPLEMENTAL DATA — 810

GENERATE
NORMALIZED DATA — 812

COMPARE TO
BIOMARKER CUT
POINTS — 814

GENERATE
REDUCED VALVES — 816

CALCULATE
COMPOSITE SCORE — 818

COMPARE TO
SCORING CUT POINTS — 820

FIG.8

24

FIG.9

1000

DATA SCORING MODEL 16,
ALL DATA OUTPUT HISTOGRAM

□ HW
□ BC

1010

FREQUENCY

BT TEST SCORING BIN

1020

| | 0 | 0.05 | 0.1 | 0.15 | 0.2 | 0.25 | 0.3 | 0.35 | 0.4 | 0.45 | 0.5 | 0.55 | 0.6 | 0.65 | 0.7 | 0.75 | 0.8 | 0.85 | 0.9 | 0.95 |
|---|---|---|---|---|---|---|---|---|---|---|---|---|---|---|---|---|---|---|---|---|
| SPECIFICITY | 13% | 23% | 41% | 51% | 60% | 73% | 85% | 90% | 91% | 95% | 95% | 98% | 100% | 100% | 100% | 100% | 100% | 100% | 100% | 100% |
| FALSE NEGATIVE | 0% | 0% | 0% | 0% | 0% | 0% | 0.6% | 2% | 2% | 2% | 2% | 3% | 4% | 7% | 7.5% | 9% | 14% | 20% | 28% | 34% |
| SENSITIVITY | 100% | 100% | 100% | 100% | 100% | 100% | 99% | 98% | 98% | 98% | 98% | 97% | 95% | 93% | 92.50% | 90% | 85% | 79% | 71% | 66% |
| FALSE POSITIVE | 86% | 76% | 58% | 48% | 40% | 26% | 15% | 10% | 8% | 5% | 5% | 2% | 0% | 0% | 0.0% | 0% | 0% | 0% | 0% | 0% |

FIG.10

Europäisches
Patentamt

European
Patent Office

Office européen
des brevets

**EUROPEAN SEARCH REPORT**

Application Number

EP 17 19 2958

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 6 714 925 B1 (BARNHILL STEPHEN [US] ET AL) 30 March 2004 (2004-03-30) <br> * the whole document * <br> * column 10, paragraph 1 * <br> * column 13, line 41 - column 22, line 46 * <br> * column 26, line 44 - column 35, line 39 * <br> * figures 3,4,6,10 * <br> * whole document, in particular fig. 4, 31, col. 19.-col. 20, col. 32, 50, example 2 * | 1-12 | INV. <br> G06F19/24 <br> G01N33/48 <br> C12Q1/00 |
| A | GOLUB T R ET AL: "Molecular classification of cancer: Class discovery and class prediction by gene expression monitoring", <br> SCIENCE, AMERICAN ASSOCIATION FOR THE ADVANCEMENT OF SCIENCE, WASHINGTON, DC; US, <br> vol. 286, no. 5439, <br> 15 October 1999 (1999-10-15), pages 531-537, XP002207658, <br> ISSN: 0036-8075, DOI: 10.1126/SCIENCE.286.5439.531 <br> * the whole document * | 1-12 | |
| A | US 2005/176057 A1 (BREMER TROY [US] ET AL) 11 August 2005 (2005-08-11) <br> * paragraph [0021] - paragraph [0030] * <br> * paragraph [0330] - paragraph [0403] * <br> * whole doc, in particular claim 8 * | 1-12 | TECHNICAL FIELDS SEARCHED (IPC) <br><br> G06F |

The present search report has been drawn up for all claims

| Place of search | Date of completion of the search | Examiner |
|---|---|---|
| Munich | 13 March 2018 | Lüdemann, Susanna |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

................................................................
& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 17 19 2958

This annex lists the patent family members relating to the patent documents cited in the above-mentioned European search report.
The members are as contained in the European Patent Office EDP file on
The European Patent Office is in no way liable for these particulars which are merely given for the purpose of information.

13-03-2018

| Patent document cited in search report | | Publication date | Patent family member(s) | | Publication date |
|---|---|---|---|---|---|
| US 6714925 | B1 | 30-03-2004 | US 6714925 B1 | | 30-03-2004 |
| | | | US 6882990 B1 | | 19-04-2005 |
| | | | US 2005165556 A1 | | 28-07-2005 |
| | | | US 2008033899 A1 | | 07-02-2008 |
| | | | US 2008059392 A1 | | 06-03-2008 |
| | | | US 2010256988 A1 | | 07-10-2010 |
| US 2005176057 | A1 | 11-08-2005 | NONE | | |

EPO FORM P0459

For more details about this annex : see Official Journal of the European Patent Office, No. 12/82